# EUROPÄISCHE PATENTANMELDUNG

(11) **EP 1 363 124 A1**
(43) Veröffentlichungstag der Anmeldung: **19.11.2003**
(21) Anmeldenummer: 03010463.2
(22) Anmeldetag: 09.05.2003
(51) Int. Cl.: G01N 33/542, G01N 33/58, G01N 33/543, C12Q 1/68, G01N 21/64

(54) **Verfahren zum Nachweis eines Analyts mit einem elasto-optischen Biosensor**

(30) Priorität: 15.05.2002 DE 10221792
(71) Anmelder: Stiftung Caesar Center of Advanced European Studies and Research, 53175 Bonn (DE)
(72) Erfinder: Hoffmann, Daniel, Dr. rer.nat., 53229 Bonn (DE); Moske, Michael, PD Dr. rer.nat., 53359 Rheinbach (DE)
(74) Vertreter: Schrooten, Rolf, Dipl.-Ing.

(57) **Zusammenfassung**

Verfahren zum qualitativen und/oder quantitativen Nachweis eines Analyten 6 in einer Lösung,
- wobei auf einer Unterlage 1 ein erstes Chromophor 2 aufgebracht ist,
- wobei das erste Chromophor 2 mit elektromagnetischer Strahlung 8 bestrahlt wird, daraus eine Wellenlänge λ absorbiert und Fluoreszenzlicht der Wellenlänge λ₁ 11 abgeben kann,
- wobei ein zweites Chromophor 3 über eine elastische Kopplung 4 an das erste Chromophor 2 gebunden ist,
- wobei zwischen den Chromophoren 2, 3 ein Fluoreszenz-Resonanz-Energie Transfer (FRET) möglich ist,
- wobei der Fluoreszenzakzeptor insbesondere nach strahlungslosem Übergang Fluoreszenzlicht der Wellenlänge λ₂ 12 abgibt,
- wobei das zweite Chromophor 3 mit einem Liganden 5 verbunden ist, an den der Analyt 6 koppelt,
- wobei Unterlage und Analytlösung relativ zueinander bewegt werden, um die elastische Kopplung 4 der Chromophore zu strecken,
- wobei während der Bewegung die Fluoreszenzstrahlung 11, 12 bei den Wellenlängen λ₁ und λ₂ beobachtet wird und
- wobei aus den Intensitäten der Fluoreszenzstrahlung 11, 12 bei den Wellenlängen λ₁ und λ₂ auf die Art und/oder die Menge des Analyten geschlossen wird.

## Beschreibung

Die Erfindung betrifft ein Verfahren zum qualitativen und/oder quantitativen Nachweis eines in Lösung befindlichen insbesondere biologischen Analyten. Die Erfindung betrifft zudem einen Sensor, insbesondere zur Umsetzung des Verfahrens.

Zweck derartiger Biosensoren ist die spezifische Detektion von Biomolekülen. Es gibt eine Reihe von wissenschaftlich und wirtschaftlich wichtigen Feldern, in denen Biosensoren zu diesem Zweck eingesetzt werden. Dazu gehören zum Beispiel die medizinische Diagnostik, die Umweltdiagnostik, die Entwicklung pharmazeutischer Wirkstoffe oder die Überwachung von industriellen biotechnologischen Prozessen. Ein Paradebeispiel für die wissenschaftliche und wirtschaftliche Bedeutung von Biosensoren sind der GeneChip® von Affymetrix® und verwandte Produkte, die eine immer größere Verbreitung in der akademischen und industriellen Forschung finden.

Die Anforderungen an solche Biosensoren sind dabei vielfältig. Erwünscht ist üblicherweise eine hohe Sensitivität und Spezifität bei kleinen Analytmengen und hohem Meßdurchsatz, eine hohe Robustheit und häufig auch die Möglichkeit zur Miniaturisierung und zur Integration in Arrays. Außerdem ist es günstig, wenn die zu detektierenden Biomoleküle nicht, wie bekannt, markiert werden müssen mit Fluorophoren oder mit radioaktiven Isotopen.

Ein verbreiteter, wirtschaftlich erfolgreicher Sensortyp basiert auf dem Effekt der Surface Plasmon Resonance (SPR). Damit lassen sich vielfältige Messungen durchführen bis hin zur Bestimmung von Bindungskinetiken von Biomolekülen. Typischerweise lassen sich Proteinmengen bis hinab zum pM-Bereich messen.

Proteine, die nur in geringen Konzentrationen vorkommen, können also auf diese Weise nicht detektiert werden. Deutlich empfindlicher ist eine Variante des SPR-Verfahrens, die außerdem die Fluoreszenz des Analyten ausnutzt. Nachteil dieser Variante ist die Notwendigkeit der Fluoreszenzmarkierung des Analyten.

Fluoreszenzmarkierungen sind auch bei dem erwähnten GeneChip ®, einem verbreiteten Verfahren mit einem relativ hohen Integrationsgrad, notwendig. Bei diesem Verfahren läßt sich ein ganzes Genom sich auf einem Array unterbringen. Die Empfindlichkeit dieses Verfahrens wird erhöht durch Amplifikation des Nukleinsäureanalyten über die Polymerase Chain Reaction (PCR). Das Verfahren ist damit sehr empfindlich. In dieser Form ist das Verfahren aber eingeschränkt auf Nukleinsäuren als Analyten. Bei der Verwendung elektro-chemischer Sensoren müssen dem eigentlichen Analyten meist noch weitere Stoffe zugesetzt werden. In Abhängigkeit von der Konzentration der eigentlich interessierenden Moleküle im Analyten kommt es dann bei Reaktionen der Zusatzstoffe zur Freisetzung elektrischer Ladungsträger, die als Strom detektiert werden. In anderer Ausführung werden zusätzlich die dielektrischen Eigenschaftsänderungen des Meßsystems unter Anwendung kapazitiver Meßverfahren detektiert oder Impedanzspektroskopie angewendet.

Ein weiterer verbreiteter Sensortyp ist die Quarz-Mikrowaage, bei der ein wichtiger Vorteil die kompakte Bauweise im Vergleich zu SPR-Systemen ist. In ihrer Empfindlichkeit vergleichbar mit SPR-Sensoren sind Surface Acoustic Wave (SAW) Mikrowaagen, die allerdings vor allem für Messungen in der Gasphase eingesetzt werden. In der flüssigen Phase, die für viele Fragestellungen die relevantere ist, leidet die Detektion mit SAW-Sensoren unter der starken Dämpfung durch die Flüssigkeit.

Massenspektrometrische Verfahren wie die Surface Enhanced Laser Desorption/lonization (SELDI) kommen zwar ohne Markierungen aus und die Empfindlichkeit solcher Verfahren reicht unter günstigen Umständen bis in den attomol-Bereich. Andererseits ist der apparative Aufwand vergleichsweise hoch, der bisher mögliche Integrationsgrad noch gering und eine Quantifizierung des Analyten schwierig.

Aus dem Stand der Technik sind somit einige Biosensortypen bekannt. In der Praxis ist es aber bisher nicht gelungen einen Biosensor zu entwickeln, der gleichzeitig und in hohem Masse allen genannten Anforderungen genügt.

Aufgabe der vorliegenden Erfindung ist es daher ein Verfahren zum Nachweis eines in Lösung befindlichen insbesondere biologischen Analyten zu schaffen, das sich einfach und mit kostengünstigen Mitteln umsetzen läßt, das die Möglichkeit zur Miniaturisierung und zur Integration in Arrays bietet und das bei Verzicht auf Marker eine hohe Sensitivität und Spezifität bei kleinen Analytmengen, einen hohen Meßdurchsatz und eine hohe Robustheit aufweist. Zudem ist es die Aufgabe, einen Sensor, speziell einen Biosensor, zur Umsetzung des Verfahrens zu schaffen.

Diese Aufgabe wird durch ein Verfahren mit den kennzeichnenden Merkmalen des Anspruch 1 und den Sensor nach Anspruch 10 gelöst.

Der Grundgedanke der Erfindung liegt darin, in Folge einer Bindung von Analytmolekülen an Liganden eine Änderung der Kräfte auf ein am Liganden befestigtes elastisches Molekül hervorzurufen, das mechanisch angeregt wird, wobei diese Änderung optisch detektiert wird als Änderung des Fluorescence Energy Resonant Transfer (FRET) zwischen zwei Chromophoren in diesem elastischen Molekül. Die Kräfte entstehen durch eine Relativbewegung zwischen der Analytlösung und der Unterlage, auf der die elastischen Moleküle gebunden sind und bewirken damit eine vom Analyten abhängige Streckung der elastischen Verbindung zwischen den beiden Chromophoren und führen damit zu einer Vergrößerung von deren Abstand zueinander. Dabei wird das System vorteilhafter Weise derart konzipiert, daß die Chromophore etwa im Abstand des Förster Radius über die elastische Verbindung koppeln.

Auf diese Änderung des Abstandes reagiert der FRET insofern sehr sensibel, als bei vergrößertem Abstand nur noch wenige Übergänge zwischen den Chromophoren stattfinden können. Die Fluoreszenzstrahlung des Systems wird dann fast nur noch von dem durch äußere Strahlung angeregten Chromophor emittiert, was sich in einer Änderung der Verhältnisse der Intensitäten beider Fluoreszenzwellenlängen ausdrückt. Ein durch Analyt belastetes System reagiert somit meßbar anders als ein unbelastetes. Ein qualitativer und insbesondere ein quantitativer Nachweis des Analyten ist somit möglich.

Das System kann derart konzipiert sein, daß die beiden Fluoreszenzwellenlängen ungleich oder gleich sind. Bei ungleichen Wellenlängen kann eine zeitgleiche Aufnahme von deren Intensitäten erfolgen. Eine Konstellation, bei der die Chromophore mit zeitverzögertem Transfer auf gleicher Wellenlänge fluoreszieren, würde insbesondere die Verwendung gepulster Strahlung bedingen. Die Aufnahme der Intensitäten der von Donor und Akzeptor entsprechend zeitverzögert emittierten Strahlung könnte mit einem Lock-In-Verfahren erfolgen.

Da erfindungsgemäß Fluoreszenzlicht optisch detektiert wird, bietet der Biosensor die für solche Fluoreszenzverfahren typische hohe Sensitivität. Im Gegensatz zu herkömmlichen Verfahren ist eine Fluoreszenzmarkierung des Analyten überflüssig.

Wie dargelegt wird FRET genutzt, der innerhalb bestimmter elastischer Moleküle, nachfolgend "Federn" genannt, stattfindet. Diese Moleküle sind fest mit einer Unterlage verbunden. Dabei wird die Eigenschaft genutzt, daß die Federn, nach optischer Anregung in einem vorgegebenen Frequenzbereich, in zwei verschiedenen anderen Frequenzbereichen Fluoreszenzlicht emittieren können, wobei das Verhältnis der beiden Emissions-Intensitäten sehr empfindlich von der Form der Federn, insbesondere von der Länge der mechanischen Streckung der Federn, abhängt.

Von besonderem Vorteil ist, daß der erfindungsgemäße Biosensor miniaturisiert, in Arrays hoher Dichte integriert und mit geringen Probenmengen betrieben werden kann. Ein Vorteil bei der Integration und Miniaturisierung liegt in der Eigenschaft, daß die nötige optische und mechanische Anregung des Substrats global erfolgen kann, während es leicht möglich ist, die optische Detektion der molekularen Reaktionen lokal aufgelöst durchzuführen. Die Detektion ist nicht beschränkt auf Biomoleküle bestimmter Klassen, solange das der Detektion vorangehende Bindungsereignis zu einer meßbaren Änderung der an den Federn wirkenden Kräften führt. Der erfindungsgemäße Biosensor kann für Messungen in einer Reihe von Intervallen von Analytkonzentrationen konfiguriert werden.

Schließlich lassen sich mit dem erfindungsgemäßen Biosensor eine Reihe unterschiedlicher Messungen durchführen. So sind zum Beispiel Bestimmungen der Konzentration von Analytmolekülen oder Messungen der Kinetik zwischen Liganden und Analytmolekülen möglich.

Der erfindungsgemäße Biosensor läßt sich einsetzen, um Wechselwirkungen zwischen verschiedenen Wechselwirkungspartnern zu analysieren. Als Paare von Liganden beziehungsweise Analytmolekülen kommen beispielsweise in Betracht: kleine Moleküle und Makromoleküle, einsträngige Nukleinsäuren und eventuell hybridisierende andere einsträngige Nukleinsäuren, Nukleinsäuren oder Peptide und Makromoleküle. Interessant für in vivo-Untersuchungen sind zum Beispiel Wechselwirkungen zwischen kleinen Molekülen oder Proteinen als Liganden und membranständigen Rezeptoren als Analytmolekülen.

Die molekularen Federn sind vorteilhafter Weise folgendermaßen aufgebaut: Über eine Gruppe wird die Feder auf der Unterlage immobilisiert zum Beispiel über eine kovalente chemische Bindung oder eine sonstige starke Bindung, wie sie zwischen einem Biotinylgruppe und Streptavidin bekannt ist. Zudem weisen die Federn einen Fluoreszenzdonor und einen darauf abgestimmten Fluoreszenzakzeptor auf. Dabei sind die Größe der Feder, die Position und die Art der beiden Fluorophore so gewählt, daß im entspannten Zustand der Feder ein Förster Transfer mit deutlich höherer Wahrscheinlichkeit vom Donor zum Akzeptor stattfinden kann, als im gedehnten Zustand der Feder. Die Federn weisen einen elastischen Teil auf, der durch Polymermoleküle realisiert werden kann, deren entropische Elastizität genutzt wird, oder der durch Nanotubes realisiert werden kann, deren Biege-Elastiztät genutzt wird. Am dem Ende der Feder, das dem Immobilisierungsende gegenüber liegt, ist ein beliebiger Ligand gekoppelt. Diese Kopplung ist kovalent oder über eine sonstige starke Bindung realisiert. Es müssen nicht beide Fluorophore Teil der Feder sein. Es kann auch ein Fluorophor am Fuß der Feder direkt auf der Unterlage gebunden werden.

Das System hat zudem eine oder mehrere Unterlagen, auf der oder denen die Federn immobilisiert sind. Die Federn haben zueinander auf der Unterlage einen Abstand, der groß ist gegenüber dem Abstand zwischen den beiden Fluorophoren innerhalb einer Feder. Falls notwendig wird die Oberfläche der Unterlage so strukturiert, daß die Federn etwas erhöht auf der Unterlage angebracht sind. Damit wird erreicht, daß die Federn von der Bewegung des Mediums in ausreichendem Maße erfaßt werden. Die Unterlagen werden bewegt mittels einer Vorrichtung, die eine insbesondere periodische Relativbewegung zwischen Unterlage und flüssigem Medium erzeugt. Es kann auch vorteilhaft sein, eine transparente Unterlage zu wählen oder als Unterlage eine transparente Kapillare zu wählen.

Zudem wird eine Lichtquelle eingesetzt, die spezifisch die Donoren anregt. Diese Lichtanregung kann gegebenenfalls gepulst erfolgen, um über eine Lock-In-Verstärkung von einem Strahlungsuntergrund zu differenzieren. Ein optischer Detektor detektiert sowohl die Fluoreszenz der Donoren wie auch die Fluoreszenz der Akzeptoren. Das System weist zudem eine Steuer- und Auswerteelektronik auf, die die Fluoreszenzsignale verknüpft mit einem Signal, das der periodischen Relativbewegung entspricht und damit die Messung der Fluoreszenzantwort im Lock-In-Verfahren erlaubt. Die optische Anregung kann auch über Oberflächenplasmonen erfolgen, wobei diese Oberflächenplasmonen durch Lichteinstrahlung über die Unterseite der transparenten Unterlage erzeugt werden. Durch Verwendung von Oberflächenplasmonen kann die Fluoreszenzausbeute und das Signal/Rauschverhältnis noch gesteigert werden. Die Emission des Lichts für die optische Anregung und die optische Detektion kann auf verschiedenen Seiten der Unterlage erfolgen.

Es sind verschiedene Ausführungsbeispiele des Sensors möglich: So lassen sich die einzelnen Komponenten des Sensors auf verschiedene Arten realisieren. Insbesondere durch die Art der Unterlage und durch die Art der Erzeugung der Relativbewegung zwischen Unterlage und Umgebung lassen sich unterschiedliche Anwendungsfelder erschließen, wie nicht abschließend an den folgenden Beispielen beschrieben wird:

Elasto-optischer Biosensor integriert in Mikrofluidik:

Die Federn werden in einer lichtdurchlässigen Kapillare immobilisiert, durch die Analytlösung gepumpt wird. Das Pumpen erfolgt dabei nicht gleichmäßig, sondern gepulst. Die Korrelation der Pulse mit den Fluoreszenzsignalen ermöglicht die Nutzung von Lock-in-Verfahren zur Erhöhung des Signal-Rausch-Verhältnisses. Die Kapillare kann Teil eines komplexeren analytischen on-line Systems sein, in dem vor oder nach der Kapillare andere Trenn- oder Analyse-Schritte durchlaufen werden (zum Beispiel Elektrospray-Massenspektrometrie).

Elasto-optischer Biosensor auf Quarz-Oszillator:

Die Relativbewegung wird durch einen geeigneten Quarz-Oszillator erzeugt, der piezoelektrisch zu Schwingungen angeregt wird. Nach diesem Prinzip läßt sich auch leicht ein Array von Sensorelementen realisieren, wobei jedes dieser Elemente Federn enthält, die mit einem anderen Liganden bestückt sind. So können bei globaler mechanischer Anregung parallel und auf kleinem Raum viele verschiedene Ligand-Analyt-Wechselwirkungen gleichzeitig gemessen werden.

Elasto-optischer Biosensor für in vivo Anwendungen:

Die Unterlagen, auf denen die Federn immobilisiert werden, können auch kleine magnetische Partikel sein. Die Relativbewegung wird dann durch ein hochfrequentes oder gepulstes magnetisches Feld erzeugt. Diese Technik erlaubt Messungen in vivo. Beispielsweise können damit Wechselwirkungen zwischen Liganden und membranständigen Proteinen auf Zelloberflächen in Zellkulturen untersucht werden.

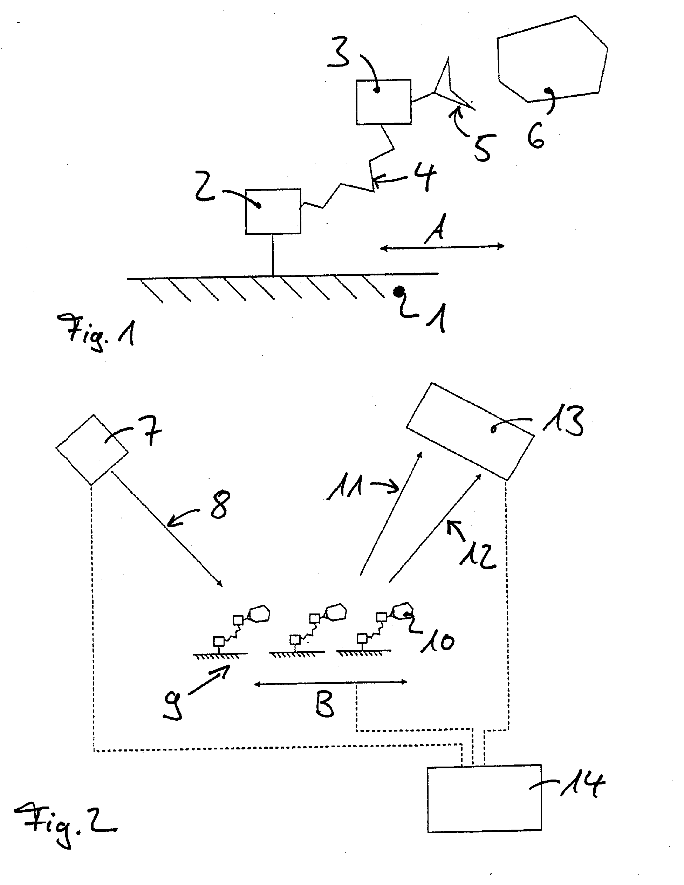

Ein Ausführungsbeispiel der Erfindung ist im folgenden anhand der Figuren 1 und 2 näher beschrieben. Es zeigen:
- **Figur 1**: das Funktionsprinzip eines erfindungsgemäßen Sensors und
- **Figur 2**: das Meßprinzip mit einem solchen Sensor.

In der Figur 1 ist das Funktionsprinzip des erfindungsgemäßen Sensors gezeigt. Auf einer insbesondere goldbeschichteten Unterlage 1, die einer periodischen Relativbewegung (Pfeil A) zur Umgebung ausgesetzt ist, ist eine molekulare Feder immobilisiert. Die Feder weist ein erstes Fluorophor 2 und ein zweites Fluorophor 3 auf, wobei in diesem Falle das erste Fluorophor 2 ein Fluoreszenzdonor und das zweite Fluorophor 3 ein dazu passender Fluoreszenzakzeptor ist. Die beiden Fluorophore sind verbunden über ein elastisches Element 4. Am Ende der Feder ist ein Ligand 5 angekoppelt, dessen Bindung an ein Analytmolekül 6 untersucht werden soll. Es ist vorteilhaft, wenn die Unterlage eine Oberflächenstrukturierung besitzt, die die Federn in die Analytlösung hinragen läßt, so daß die Federn von der Bewegung der Flüssigkeit erfaßt werden.

Figur 2 stellt das Meßprinzip mit einem erfindungsgemäßen Sensors dar. Eine Lichtquelle 7 emittiert Licht 8 der Wellenlänge λ, mit dem die Fluoreszenzdonoren angeregt werden. Je nach Auslenkungen der Federn 9, die bei Relativbewegung (Pfeil B) geschieht und die abhängt von der Art der an den Liganden hängenden Analytmoleküle 10, verändert sich das Verhältnis der Intensitäten zwischen Fluoreszenzlicht 11 der Wellenlänge λ₁, das direkt vom Donor emittiert wird, und Fluoreszenzlicht 12 der Wellenlänge λ₂, das über Förster-Transfer vom Donor auf den Akzeptor übertragen wird und dann vom Akzeptor emittiert wird. Das Licht der Wellenlängen λ₁ und λ₂ wird in einem Detektor 13 registriert. Eine zeitliche Pulsabfolge der Lichtemission, die Relativbewegung von Unterlage und Umgebung, sowie die detektierten Lichtintensitäten werden in einem Lock-in-Verstärker 14 korreliert, um damit das Verhältnis vom Signal zum Rauschen zu erhöhen.

Nachfolgend wird eine Folge typischer Verfahrensschritte beschrieben:

Zunächst wird der Analyt mit dem Biosensor zusammengebracht. Je nach Ausführung wird der Analyt dabei auf den Sensor gebracht, diesem über eine Fluidikvorrichtung zugeführt oder es werden Teile des Sensors in den Analyten eingebracht. Wesentlich ist, daß der Analyt die Federn umgibt oder umspült. Während einer Equilibrierungsphase haben Analytmoleküle die Gelegenheit zur spezifischen Bindung an die Liganden an den Federenden. Nun werden der Analyt und die Unterlage der Federn periodisch gegeneinander bewegt. Dabei werden Amplitude und Frequenz der Bewegung so gewählt, daß sich die Kräfte auf die Federn ohne gebundene Analytmoleküle und mit gebundenen Analytmolekülen deutlich unterscheiden und damit auch die Auslenkungen der Federn in diesen beiden Fällen.

Gleichzeitig wird die Fluoreszenz des Donors durch Lichteinstrahlung spezifisch angeregt, ohne den Akzeptor durch diese Lichteinstrahlung direkt anzuregen. Die Anregung des Akzeptors findet hingegen indirekt durch Förster-Transfer vom Donor zum Akzeptor statt. Im Falle einer wenig oder nicht ausgelenkten Feder sind Donor und Akzeptor nahe beieinander und der Förster-Transfer findet mit einer hohen Wahrscheinlichkeit statt. Damit wird eine erhöhte Fluoreszenz-Intensität im Frequenzbereich der Emissiondes Akzeptors detektiert und eine verringerte Intensität der Emission des Donors. Falls ein Analytmolekül an den Liganden gebunden hat erhöhen sich die Kräfte (Reibung, Trägheit), die aufgrund der periodischen Bewegung auf die Feder wirken. Wenn die Krafterhöhung groß genug ist, wird die Feder soweit ausgelenkt, daß der Abstand zwischen Donor und Akzeptor keinen Förster-Transfer mehr erlaubt. Dann wird vor allem der Donor mit erhöhter Intensität und der Akzeptor mit verringerter Intensität fluoreszieren. Das Verhältnis der Intensitäten der beiden Fluoreszenzen verschiebt sich also bei Vergrößerung der Kräfte hin zur Fluoreszenz des Donors.

Die Fluoreszenz wird detektiert. Zur Verbesserung des Signal zu Rausch Verhältnisses wird das Fluoreszenzsignal dabei korreliert mit der mechanischen Anregung der Bewegung zwischen Feder und Umgebung und der optischen Anregung der Fluoreszenz. Die relative Änderung der Intensitäten der Fluoreszenz von Donor und Akzeptor im Vergleich zu Kontrollversuchen, die keine spezifisch bindenden Analytmoleküle enthalten, gibt Aufschluß über die Änderung der Kräfte auf die Federn als Folge der Bindung von Analytmolekülen, und damit über die Größe und die Art dieser Analytmoleküle sowie über deren Konzentration.

## Patentansprüche

1. Verfahren zum qualitativen und/oder quantitativen Nachweis eines Analyten (6) in einer Lösung,
- wobei auf einer Unterlage (1) ein erstes Chromophor (2) aufgebracht ist,
- wobei das erste Chromophor (2) mit elektromagnetischer Strahlung (8) bestrahlt wird, daraus eine Wellenlänge λ absorbiert und Fluoreszenzlicht der Wellenlänge **λ**₁ (11) abgeben kann,
- wobei ein zweites Chromophor (3) über eine elastische Kopplung (4) an das erste Chromophor (2) gebunden ist,
- wobei zwischen den Chromophoren (2,3) ein Fluoreszenz-Resonanz-Energie Transfer (FRET) möglich ist,
- wobei der Fluoreszenzakzeptor insbesondere nach strahlungslosem Übergang Fluoreszenzlicht der Wellenlänge λ₂ (12) abgibt,
- wobei das zweite Chromophor (3) mit einem Liganden (5) verbunden ist, an den der Analyt (6) koppelt,
- wobei Unterlage und Analytlösung relativ zueinander bewegt werden, um die elastische Kopplung (4) der Chromophore zu strecken,
- wobei während der Bewegung die Fluoreszenzstrahlung (11,12) bei den Wellenlängen λ₁ und λ₂ beobachtet wird und
- wobei aus den Intensitäten der Fluoreszenzstrahlung (11,12) bei den Wellenlängen λ₁ und λ₂ auf die Art und/oder die Menge des Analyten geschlossen wird.

2. Verfahren nach Anspruch 1,
**dadurch gekennzeichnet, daß** die Chromophore derart gewählt sind, daß die Wellenlängen λ₁ und λ₂ der Fluoreszenzstrahlung (11,12) unterschiedlich sind.

3. Verfahren nach Anspruch 1,
**dadurch gekennzeichnet, daß** die Chromophore derart gewählt sind, daß die Wellenlängen λ₁ und λ₂ der Fluoreszenzstrahlung (11,12) gleich sind.

4. Verfahren nach einem der vorherigen Ansprüche,
**dadurch gekennzeichnet**,daßdieMessungder Fluoreszenzintensität des Donors und des Akzeptors zeitaufgelöst nach einer gepulsten Anregung des Donors erfolgt.

5. Verfahren nach einem der vorherigen Ansprüche,
**dadurch gekennzeichnet, daß** die elastische Verbindung (4) der Chromophore (2,3) im ungestreckten Zustand einen deutlich stärkeren FRET zeigt als im gestreckten Zustand.

6. Verfahren nach einem der vorherigen Ansprüche,
**dadurch gekennzeichnet**,daßbeiderBestimmungvon Masse und Konzentration als Bewegung eine periodische Bewegung insbesondere eine Schwingung verursacht wird, wobei eine Auslenkung gewählt wird, die nicht zur Zerstörung der elastischen Verbindung führt.

7. Verfahren nach einem der vorherigen Ansprüche,
**dadurch gekennzeichnet**,daßbeiderBestimmungder Verbindungsstärke eine kontrollierte Bewegung verursacht wird, die zur Zerstörung der elastischen Verbindung führt.

8. Verfahren nach einem der vorherigen Ansprüche,
**dadurch gekennzeichnet, daß** die Relativbewegung zwischen Analytlösung und Unterlage (1) über einen Schwingquarz, ein magnetisches Wechselfeld oder eine periodisch arbeitende Pumpe angeregt wird.

9. Verfahren nach einem der vorherigen Ansprüche,
**dadurch gekennzeichnet**,daßdieoptischeAnregungüber Oberflächenplasmonen erfolgt, wobei diese Oberflächenplasmonen durch Lichteinstrahlung über die Unterseite einer transparenten Unterlage erzeugt werden.

10. Sensor, insbesondere zur Durchführung des Verfahrens nach einem der vorherigen Ansprüche,
**gekennzeichnet durch,**
- elastische Moleküle ("Federn") aufweisend einen Teil, über den die Moleküle an einer oder mehreren Unterlagen (1) fest gekoppelt sind, einem ersten Chromophor (2), einem elastischen Verbindungsstück (4) zu einem zweiten Chromophor (2), sowie einem fest an das zweite Chromophor (2) gekoppelten Liganden (5),
- eine Bewegungsvorrichtung zur Erzeugung einer Relativbewegung zwischen Unterlage (1) und Analytlösung,
- einer Lichtquelle (7) zur Aussendung von Licht (8), das eines der beiden Chromophore (2,3) spezifisch anregt,
- einem optischen Detektor (13), der die Fluoreszenz von Donoren und Akzeptoren detektiert und
- einer Vorrichtung (14), die die Fluoreszenzsignale verknüpft einerseits mit einem Signal, das der insbesondere periodischen Relativbewegung entspricht, und andererseits einem Signal, das dem optischen Anregungssignal entspricht und damit die Messung der Fluoreszenzantwort insbesondere im Lock-In-Verfahren erlaubt.

11. Sensor nach Anspruch 10,
**dadurch gekennzeichnet, daß** die Unterlage (1)eine Oberflächenstrukturierung besitzt, die die Federn in die Analytlösung hineinragen läßt, so daß die Federn von der Bewegung der Flüssigkeit erfaßt werden.

12. Sensor nach Anspruch 10 oder 11,
**dadurch gekennzeichnet, daß** die Unterlage (1) transparent ist.

13. Sensor nach einem der Ansprüche 10 bis 12,
**dadurch gekennzeichnet,daß** die Unterlagen(1) magnetische Partikel sind.

14. Sensor nach einem der Ansprüche 10 bis 13,
**dadurch gekennzeichnet, daß** die Feder ein Polymer oder ein Nanotube aufweist.

15. Sensor nach einem der Ansprüche 10 bis 14,
**dadurch gekennzeichnet, daß** die Unterlage (1)auf regelmässige Weise in Felder unterteilt ist ("Array"), wobei jedes Feld mit einer jeweils anderen Art von Federn versehen ist.

16. Sensor nach einem der Ansprüche 10 bis 15,
**dadurch gekennzeichnet, daß** der Detektor (14) ein Charge Coupled Device ist, wobei insbesondere die Detektion jedes Feldes des Arrays simultan durch einen entsprechenden Teil des Charge Coupled Device erfolgt.

17. Sensor nach einem der Ansprüche 10 bis 16,
**dadurch gekennzeichnet,**daßdieLigandenorganische Moleküle, Einzelstrang-Nukleinsäuren, Doppelstrang-Nukleinsäuren, Peptide und/oder Proteine sind.
